(19) Europäisches Patentamt
European Patent Office
Office européen des brevets

(11) EP 1 657 263 B1

(12) **EUROPEAN PATENT SPECIFICATION**

(45) Date of publication and mention of the grant of the patent:
**02.07.2008 Bulletin 2008/27**

(21) Application number: **04747593.4**

(22) Date of filing: **15.07.2004**

(51) Int Cl.:
***C08F 220/04*** *(2006.01)*

(86) International application number:
**PCT/JP2004/010127**

(87) International publication number:
**WO 2005/007714 (27.01.2005 Gazette 2005/04)**

(54) **POLYMER SUSTAINEDLY RELEASING AMINO ACID DERIVATIVE, COSMETIC AND FIBER CONSTRUCT CONTAINING THE POLYMER, METHOD OF PRODUCING THE SAME AND METHOD OF REGENERATING THE SAME**

GLEICHMÄSSIG HINHALTEND EIN AMINOSÄUREDERIVAT FREISETZENDES POLYMER, KOSMETIKUM UND FASERKONSTRUKT MIT DEM POLYMER, HERSTELLUNGSVERFAHREN DAFÜR UND REGENERATIONSVERFAHREN DAFÜR

POLYMERE A LIBERATION SOUTENUE DE DERIVES D'ACIDES AMINES, PRODUIT COSMETIQUE ET PRODUIT DE SYNTHESE A BASE DE FIBRES CONTENANT CE POLYMERE, PROCEDE DE PRODUCTION DE CE POLYMERE ET PROCEDE POUR LE REGENERER

(84) Designated Contracting States:
**AT BE BG CH CY CZ DE DK EE ES FI FR GB GR HU IE IT LI LU MC NL PL PT RO SE SI SK TR**

(30) Priority: **18.07.2003 JP 2003276373**

(43) Date of publication of application:
**17.05.2006 Bulletin 2006/20**

(73) Proprietors:
- **JAPAN EXLAN COMPANY LIMITED**
**Osaka-shi**
**Osaka 530-0004 (JP)**
- **Toyo Boseki Kabushiki Kaisha**
**Kita-ku, Osaka-shi**
**Osaka 530-8230 (JP)**

(72) Inventors:
- **IENO, Masao**
**Okayama-shi,**
**Okayama 704-8194 (JP)**
- **FURUTA, Kiyonori,**
**c/o AJINOMOTO CO. INC.**
**Kawasaki-shi,**
**Kanagawa 210-8681 (JP)**

(74) Representative: **Strehlke, Ingo Kurt et al**
**Gesthuysen, von Rohr & Eggert**
**Postfach 10 13 54**
**45013 Essen (DE)**

(56) References cited:

| | |
|---|---|
| **EP-A2- 0 761 867** | **WO-A-00/39176** |
| **WO-A1-01/12230** | **WO-A1-98/08897** |
| **JP-A- 8 301 723** | **JP-A- 10 237 126** |
| **JP-A- 2001 072 764** | **JP-A- 2002 038 375** |

- **DATABASE WPI Week 198734 Derwent Publications Ltd., London, GB; AN 1987-238756 XP002413298 -& JP 62 161711 A (SUNSTAR KK) 17 July 1987 (1987-07-17)**
- **DATABASE WPI Week 200281 Derwent Publications Ltd., London, GB; AN 2002-746163 XP002413299 -& JP 2002 284619 A (AJINOMOTO KK) 3 October 2002 (2002-10-03)**
- **DATABASE WPI Week 200035 Derwent Publications Ltd., London, GB; AN 2000-407078 XP002413300 -& JP 2000 143478 A (KAO CORP) 23 May 2000 (2000-05-23)**
- **DATABASE WPI Week 199619 Derwent Publications Ltd., London, GB; AN 1996-185467 XP002413303 -& JP 08 060547 A (SEIREN CO LTD) 5 March 1996 (1996-03-05)**
- **DATABASE WPI Week 200428 Derwent Publications Ltd., London, GB; AN 2004-297969 XP002413301 -& JP 2004 018490 A (POLA CHEM IND INC) 22 January 2004 (2004-01-22)**
- **DATABASE WPI Week 200442 Derwent Publications Ltd., London, GB; AN 2004-443881 XP002413302 -& JP 2004 123575 A (ROHTO SEIYAKU KK) 22 April 2004 (2004-04-22)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 657 263 B1

## Description

Technical Field of the Invention

The present invention relates to a polymer having a skincare characteristic and, more particularly, it relates to a polymer having an effect of supplementing a water-retaining function of horny layer and of keeping the normal skin (hereinafter, it may be referred to as a skincare effect) by supplying an amino acid derivative to the skin.

Background Art

Amino acids and amino acid derivatives such as protein are natural moisturizing factor which is inherently available in human body and have been known to have a skincare characteristic. In recent year, as a result of paying attention to such a characteristic, development of fiber products which are friendly to the skin has been carried out by applying amino acid and protein thereto.
For example, in Japanese Patent Laid-Open No. 08/060,547, a skincare product where sericin which is a protein is added to fiber product is disclosed. Said product is prepared by dipping the fiber product in an aqueous solution of sericin followed by drying but, since there is no factor for positive fixing of sericin to the fiber product, amount of the added sericin is small and, in addition, the added sericin is apt to be detached. Therefore, its skincare effect is little and is not satisfactory.
In Japanese Patent Laid-Open No. 2002/013,071, a fiber product where arginine which is an amino acid is added via a binder is disclosed. In Example 1 of said document, retaining rate of arginine after the washing for ten times is mentioned to be more than 90 % and it is mentioned that, by the use of a binder, an excellent durability against washing is achieved. However, from another viewpoint, arginine added thereto is hardly released. Thus, when a binder is used, transfer of amino acid derivative to the skin is inevitably poor and the resulting skincare fect is also little.
In order to solve such a disadvantage, addition of much more amino acid derivative may be considered. However, for such a purpose, more binder corresponding to that is to be used and, when the amount of the binder used becomes much, hand becomes hard. When it is used for a use of contacting to the skin, deterioration of hand is a big problem. Moreover, in actual use, much amount of amino acid derivative which is not released and is not participated in the skincare effect is to be added and that is not preferred in terms of economy.
In Japanese Patent Laid-Open No. 05/295,657, there is a disclosure for a method where, after an aqueous solution of amino acid is impregnated to a fiber structure, the amino acid is subjected to a cross-linking polymerization by a plasma treatment, whereby the amino acid is fixed to the fiber structure. In said method, although no binder is used, the amino acid becomes a water-insoluble polymer by a cross-linking polymerization and is firmly fixed on the surface of the fiber. Therefore, even when the fiber structure manufactured by said method is contacted to the skin, transfer of the amino acid to the skin is hardly noted as same as above and the skincare effect is not so much expected.
JP-A-62 161711 relates to a weak-acidic gel-like cosmetic where a carboxyvinyl polymer is used as a gelling agent. This document describes that, since the human skin is weak-acidic in healthy condition, a cosmetic is preferably weak-acidic, that a cosmetic requires adequate viscosity, and that the aim of the invention of this document is thus to provide a cosmetic which is weak-acidic and which has adequate viscosity. This aim is achieved by the co-use of a basic amino acid and an alkaline metal hydroxide as a neutralizing agent. In summary, this document relates to a cosmetic and describes to ionically bond a water-soluble carboxyvinyl polymer to a basic amino acid, namely with the aim to provide a cosmetic which is weak-acidic and which has adequate viscosity.
JP-A-2002 284619 relates to a method for manufacturing a cosmetic containing a specific cystine derivative and a basic amino acid. The aim of this document is to solve the problem that unpleasant odor occurs when compounding a specific cystine derivative with a basic amino acid. In this document it is described that it was found that the specific cystine derivative and the basic amino acid can be compounded together without occurring unpleasant odor by well-mixing an anionic water-soluble polymer which contains carboxyl group in a molecule with the basic amino acid so as to neutralize them in a first stage, and by mixing the specific cystine derivative represented by formula (I) as indicated in this document therewith in a second stage. In summary, this document relates to a cosmetic and describes to ionically bond an anionic water-soluble polymer which contains carboxyl group to a basic amino acid with the aim to prevent the occurrence of unpleasant odor.

Problems that the Invention is to Solve

As mentioned above, there are problems in the prior art that amino acid derivative is added in a small amount whereby it is easily detached or conversely that the amino acid derivative is hardly released even if it is able to be firmly fixed and further that hand lowers. The present invention has been carried out on the basis of the status of the prior art as such and its object is to provide a polymer which achieves an effect of supplementing the water-retaining function of

horny layer and keeping the normal skin by a gradual supplying of an amino acid derivative to the skin through moisture containing electrolyte salt such as sweat perspired from human body and to provide a cosmetic and a fiber structure con-taining said polymer. Further object of the present invention is to provide a method for the manufacture of the aforementioned polymer, cosmetic and fiber structure and a regeneration process therefore.

Means for Solving the Problems

The present inventors have carried out intensive investigations for achieving the aforementioned objects and found that, when ionic bond is adopted as a means for retaining the amino acid derivative on the polymer, the amino acid derivative is gradually released when said polymer is contacted to moisture containing an electrolytic salt whereby an excellent skincare effect is achieved and further that, when the polymer after the amino acid derivative is released is processed with an amino acid derivative solution again, it is possible to subject the amino acid derivative to an ionic bond again and to regenerate the skincare characteristic. As a result thereof, the present invention has been achieved.
Thus, the present invention relates to a sustained-release polymer for amino acid derivative as defined in Claim 1. Further, advantageous embodiments are the subject-matter of the respective dependent claims (i.e. Claims 2 to 12).
Further, the present invention relates to a method for the manufacture of the sustained-release polymer for amino acid derivative of the present invention as defined in Claim 13.
Moreover, the present invention relates to a cosmetic containing the sustained-release polymer for amino acid derivative of the present invention as defined in Claim 14.
Furthermore, the present invention relates to a fiber structure containing the sustained-release polymer for amino acid derivative of the present invention as defined in Claims 15 and 16 and a respective method for the manufacture of such fiber structure as defined in Claim 17.
Finally, the present invention relates also to a method for regeneration of the sustained-release polymer for amino acid derivative of the present invention as defined in Claim 18.
Thus, the present invention has been achieved by the following means.

(1) Sustained-release polymer for amino acid derivative, wherein a polymer containing acidic group is ionically bonded to an amino acid derivative, wherein the polymer containing acidic group has a cross-linked structure and wherein the eluting rate ($\alpha$) of the amino acid derivative when the polymer is dipped in an artificial sweat liquid is 10 % or more and is five times or more of the eluting rate ($\beta$) of the amino acid derivative when the polymer is dipped in pure water.

(2) The sustained-release polymer for amino acid derivative according to (1),
wherein the polymer is able to be regenerated when a solution of the amino acid derivative is impregnated thereinto after release of the amino acid derivative.

(3) The sustained-release polymer for amino acid derivative according to (1) or (2), wherein the amino acid derivative has the structure as shown by the following formula [I] in its molecule.

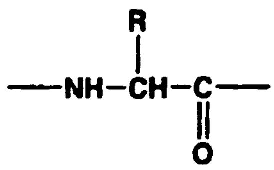

[ I ]

(R is a group having one or more basic functional group(s).)

(4) The sustaited-release polymer for amino acid derivative according to any of (1) to (3), wherein the amino acid derivative is a basic amino acid.

(5) The sustained-release polymer for amino acid derivative according to any of (1) to (4), wherein the amino acid derivative is at least one member selected from the group consisting of arginine, lysine and histidine.

(6) The sustained-release polymer for amino acid derivative according to any of (1) to (5), wherein the polymer

containing acidic group has a saturated hygroscopic degree of 20 % by weight or more under the condition of 20°C × 65 % RH.

(7) The sustained-release polymer for amino acid derivative according to any of (1) to (6), wherein the polymer containing acidic group has a carboxyl group.

(8) The sustamed-release polymer for amino acid derivative according to any of (1) to (7), wherein the polymer containing acidic group is a polymer of an acrylic acid type.

(9) The sustained-release polymer for amino acid derivative according to any of (1) to (8), wherein the cross-linked structure is formed by the reaction of nitrile group with a hydrazine type compound.

(10) The sustained-release polymer for amino acid derivative according to any of (1) to (8), wherein the cross-linked structure is formed by copolymerization of a cross-linking vinyl monomer.

(11) The sustained-release polymer for amino acid derivative according to any of (1) to (11), wherein the polymer containing acidic group is in particles.

(12) The sustained-release polymer for amino acid derivative according to any of (1) to (10), wherein the polymer containing acidic group is fibrous.

(13) A method for the manufacture of the sustained-release polymer for amino acid derivative mentioned in any of (1) to (12), characterized in that, a solution of amino acid derivative is added to a polymer containing acidic group and then the polymer is dried at 40 to 100°C.

(14) A cosmetic containing the sustained-release polymer for amino acid derivative mentioned in (11).

(15) A fiber structure containing the sustained-release polymer for amino acid derivative mentioned in (11) or (12).

(16) The fiber structure according to (15), wherein the fiber structure is selected from underwear, stomach band, supporter, mask, gloves, socks, stockings, pajama, bathrobe, towel, mat and bedclothes.

(17) A method for the manufacture of the fiber structure mentioned in (15) or (16), characterized in that, a solution of amino acid derivative is added to a material fiber structure which contains a polymer containing acidic group and then the fiber structure is dried at 40 to 100 °C.

(18) A method for regeneration of a sustained-release polymer for amino acid derivative, characterized in that, a solution of amino acid derivative is added to the sustained-release polymer for amino acid derivative mentioned in any of (1) to (12) or to the fiber structure mentioned in (15) or (16) in which amount of the amino acid derivative bonded thereto has lowered as a result of use and then the polymer or the fiber structure is dried.

Advantages of the Invention

In the sustained-release polymer for amino acid derivative according to the present invention, ionic bond is adopted as a means for retaining the amino acid derivative on the polymer whereby the property that the amino acid derivative is able to be retained without lowering the hand and also that the amino acid derivative is gradually released therefrom is able to be achieved. The sustained-release polymer for amino acid derivative according to the present invention having such a property has an excellent skincare characteristic and is able to be used in broad areas of use such as a product contacting to the skin including cosmetic and underwear and as a material therefore.

In addition, even when an amino acid derivative is once released, the sustained-release polymer for amino acid derivative according to the present invention is able to be easily regenerated for its function by a treatment with an amino acid derivative solution again and, therefore, it is able to be utilized particularly advantageously to such a thing which is repeatedly washed such as underwear and T shirt.

Brief Description of the Drawing

Fig. 1 is a graph showing a mean value of moisture recovering rate of the skin for each of knitted cloth and unprocessed cloth of Example 9.

Best Mode for Carrying Out the Invention

The present invention will now be illustrated in detail as hereunder. The sustained-release polymer for amino acid derivative according to the present invention comprises a polymer containing acidic group and an amino acid derivative and they are connected by means of an ionic bond. The characteristic of the sustained-release polymer for amino acid derivative according to the present invention to gradually release the amino acid derivative and the characteristic thereof to regenerate are the characteristics derived from a reversible property of the ionic bond.

Thus, when an amino acid derivative which is subjected to ionic bond to the polymer containing acidic group is contacted to moisture or, particularly, to water containing an electrolyte salt such as sweat, the bond is dissociated and the amino acid derivative transfers to the skin to achieve a skincare effect. On the other hand, when an amino acid derivative solution is added to the sustained-release polymer for amino acid derivative in which the bonded amount of the amino acid derivative has lowered, ionic bond with the amino acid derivative is formed again whereby it is able to be regenerated to the original state.

Formation of ionic bond means that a strong interaction acts between the polymer containing acidic group and the amino acid derivative and, when the amino acid derivative is added, the amino acid derivative is strongly drawn to the polymer containing acid group whereby it is possible to efficiently retain large amount of amino acid derivative and it is possible to give a product having better skincare effect.

On the other hand, when an amino acid derivative is subjected to a covalent bond to the polymer or is fixed by a binder, it is not easy to transfer the amino acid derivative to the skin. Further, it is also almost impossible to regenerate it. In this regard, when it is merely attached to the polymer by a physical means, although there may be some possibilities for transfer of the amino acid derivative to the skin and for regeneration, it is just attached and interaction between the polymer and the amino acid derivative is only little whereby the amount of the amino acid which is able to be attached is also little and the skincare effect is rarely expected.

As mentioned hereinabove, the sustained-release polymer for amino acid derivative according to the present invention has a characteristic of transferring the amino acid derivative to the skin. Although it is difficult to directly evaluate the characteristic, indirect yardsticks therefore will be an eluting rate ($\alpha$) of the amino acid derivative when the polymer is dipped into an artificial sweat and an eluting rate ($\beta$) of the amino acid derivative when the polymer is dipped in pure water. As a result of investigation of the present inventors, a significant skincare effect is able to be achieved when said eluting rate ($\alpha$) upon measurement under the measuring conditions which will be mentioned later is not less than 10 % by weight, preferably not less than 30 % by weight and, more preferably, not less than 50 % by weight and the value of $\alpha/\beta$ regarding to an eluting rate ($\alpha$) of the amino acid derivative when the polymer is dipped into an artificial sweat and an eluting rate ($\beta$) of the amino acid derivative when the polymer is dipped in pure water is not less than 5 and, preferably, not less than 7.

The eluting rate ($\alpha$) of the amino acid derivative when the polymer is dipped into an artificial sweat is a value calculated by the following formula using the values measured by the following method.

$$\alpha(\%) = [(\text{Eluted amount of amino acid derivative when the polymer is dipped in artificial sweat}) \div (\text{Initial bonded amount of the amino acid derivative})] \times 100$$

With regard to the initial bonding amount (mg/g) of an amino acid derivative bonded to the sustained-release polymer for amino acid derivative, the initial bonding amount of an amino acid derivative per 1 g of the sustained-release polymer for amino acid derivative is determined by calculating from the amount of the amino acid derivative extracted by dipping 0.5 g of the sustained-release polymer for amino acid derivative in 25 mL of 0.5N aqueous solution of hydrochloric acid at 40°C for 30 minutes and from a sample moisture amount determined by a method according to weight loss on drying (dried *in vacuo* on $P_2O_5$ at 40°C for 12 hours).

With regard to an eluted amount (mg/g) of an amino acid derivative when the polymer is dipped in artificial sweat, the amount of the amino acid derivative eluted from 1 g of the sustained-release polymer for amino acid derivative is determined by calculating from the amount of the amino acid derivative eluted by dipping 0.5 g of the sustained-release polymer for amino acid derivative in 25 mL of artificial sweat at 25 °C for 0.5 hour and from the sample moisture amount determined by a method according to weight loss on drying (dried *in vacuo* on $P_2O_5$ at 40 °C for 12 hours). With regard

to the artificial sweat, the following acidic sweat liquid is prepared by referring to JIS-L-0848 and used. With regard to the artificial sweat, acidic sweat liquid and alkali sweat liquid are mentioned in JIS-L-0848 and, in the artificial sweat used in the present invention, the acidic sweat liquid is handled as a typical one thereof. With regard to the acidic sweat liquid, 5 g/L of NaCl and 2.26 g of sodium dihydrogenphosphate dodecahydrate are dissolved in water, the solution is adjusted to pH 5.5 with 0.5M aqueous solution of NaOH, then its volume is adjusted to 1 L and the product is used.

The eluting ratio (β) of the amino acid derivative when the polymer is dipped in pure water is a value calculated by the following formula using the aforementioned initial bonding amount of the amino acid derivative and the value measured by the following method.

$$\beta\ (\%) = [(\text{Eluted amount of amino acid derivative when the polymer is dipped in pure water}) \div (\text{Initial bonded amount of the amino acid derivative})] \times 100$$

With regard to an eluted amount (mg/g) of an amino acid derivative when the polymer is dipped in pure water, the amount of the amino acid derivative eluted from 1 g of the sustained-released polymer for amino acid derivative into pure water is determined by calculating from the amount of the amino acid derivative eluted by dipping 0.5 g of the sustained-release polymer for amino acid derivative in 25 mL of pure water at 25 °C for 0.5 hour and from the sample moisture amount determined by a method according to weight loss on drying (dried *in vacuo* on $P_2O_5$ at 40 °C for 12 hours). With regard to pure water, that which is manufactured by an apparatus for the manufacture of pure water (manufactured by Nippon Millipore) and has not less than 1 MΩ.cm of electric resistance, not more than 10 ppm of TOC and 7.0 ± 0.5 of pH is used.

The amino acid derivative eluted in each of the aforementioned dipping liquids is subjected to quantitative analysis using an analytical method suitable for each compound. When the amino acid derivative is a basic amino acid, the use of an amino acid analyzer is of high reliability and is simple.

It will be illustrated in order to make sure that the eluting rate of not less than 10 % by weight in the aforementioned measuring method is a desired eluting rate when the sustained-release polymer for amino acid derivative is dipped in a large amount of artificial sweat and is not the amount which is eluted in actual use. Thus, when the sustained-release polymer for amino acid derivative according to the present invention is actually used to the skin, it is not dipped in large amount of sweat but is contacted to sweat in far less amount and, therefore, the amino acid derivative is not released in large amount but is gradually transferred to the skin little by little. Actually, it is not easy to stably measure the eluted amount of an amino acid derivative when contacted to a few amount of sweat and, therefore, evaluation is carried out by the aforementioned method in the present vention.

Now the amino acid derivative concerning the present invention will be illustrated. In the present invention, the term "amino acid derivative" is used not only for the compound where amino acid or a part of functional groups in amino acid molecule is modified but also for the compound where amino acid is a constituting unit such as polypeptide, protein and hydrolyzed protein.

With regard to the amino acid derivative, any compound such as that is derived from nature and that is chemically synthesized may be essentially used so far as it is able to be subjected to ionic bond to a polymer containing acidic group. However, in view of safety to human body and economy, that derived from nature is preferred and silk protein such as fibroin and sericin, milk protein such as casein, tissue protein for skin and bone such as collagen, thermally denatured product thereof such as gelatin and amino acid such as glycine, alanine, valine, leucine, isoleucine, methionine, proline, phenylalanine, tryptophan, serine, threonine, asparagine, glutamine, tyrosine, cysteine, lysine, arginine, histidine, aspartic acid and glutamic acid may be used.

Among the aforementioned amino acid derivatives, those having the aforementioned formula [I] are more preferred. In said structure, ionic bond is able to be formed more efficiently between the basic functional group in R and the acidic group in the polymer containing acidic group. Here, examples of the basic functional group in R are amino group, guanidyl group and histidyl group. The basic functional group may be either in a free state or in a form of inorganic acid salt such as hydrochloride, sulfate, nitrate and phosphate or of organic acid salt such as citrate, succinate, p-toluenesulfonate and methanesulfonate. Examples of the amino acid derivative as such are protein such as casein, keratin and collagen and basic amino acid such as lysine, arginine and histidine.

Among them, basic amino acid has less molecular weight and is easy to make into a solution as compared with protein etc. and, therefore, it is easy to manufacture the sustained-release polymer for amino acid derivative according to the present invention and is preferred in an industrial viewpoint as well. In addition, arginine, lysine and histidine which are basic amino acids are amino acids which are present in human body and are contained in natural moisturizing factor and they are able to be advantageously used in view of the skincare effect as well.

In the present invention, it is basically possible that the more the amount of the acidic group in the polymer

containing acidic group, the more the bonding amount of the amino acid derivative and it is now easy that an amino acid derivative in an amount of as much as several tens percent is bonded which has been difficult to achieve in the conventional art using, for example, a binder. With regard to the amount of the amino acid derivative to be subjected to ionic bond to the polymer containing acidic group, it is able to be appropriately selected from a wide range depending upon the required property, use, etc. Usually however, it is preferred to be 1 to 30 % by weight, more preferably 2 to 25 % by weight and, still more preferably, 3 to 15 % by weight to the resulting sustained-release polymer for amino acid derivative. When amount of the amino acid derivative to be subjected to ionic bond is more than 30 % by weight, the cost becomes high and enhancement of the effect is not noted as well. When the amino acid derivative to be subjected to ionic bond is less than 1 % by weight, a skincare effect may not be available and that is not preferred.

Now the polymer containing acidic group concerning the present invention will be illustrated. With regard to the polymer containing acidic group concerning the present invention, it is necessary to contain an acidic group. There is no particular limitation besides the above and it is possible to use polymer of a polyester type, polymer of a polyamide type, polymer of a polyether type, polymer of a vinyl type, polymer of a cellulose type and polymer in which plural polymers are compounded.

It is preferred that the polymer containing acidic group concerning the present invention has a saturated hygroscopic degree of not less than 20 % by weight under the condition of 20 °C × 65 % RH. In that case, the hygroscopicity owned by the polymer containing acidic group and the effect for supplementing the moisture-retaining function of horny layer owned by the amino acid derivative to keep the normal skin synergistically act whereby it is now possible to achieve more excellent skincare effect. Incidentally, when the saturated hygroscopic degree under the condition of 20°C × 65 % RH is less than 20 % by weight, the synergistic effect is not able to be expected so much.

With regard to an acidic group in the polymer containing acidic group concerning the present invention, there is no particular limitation so far as it is able to form an ionic bond with an amino acid derivative and its examples are carboxyl group, sulfonic acid group and phosphoric acid group. Among them, carboxyl group is particularly preferred because it is easy to introduce into the polymer in large amount and able to form an ionic bond with the amino acid derivative in large amounts. Incidentally, ionic exchange is apt to happen and ionic bond between an acidic group and an amino acid derivative is easily formed when such an acidic group in a state where a salt such as metal salt or ammonium salt is formed is subjected to an adding treatment to the amino acid derivative.

When the polymer containing acidic group is a polymer of a vinyl type for example, the aforementioned acidic group is able to be introduced by copolymerization of a vinyl monomer containing an acidic group. Examples of the vinyl monomer containing a sulfonic acid group are vinylsulfonic acid, (meth)allylsulfonic acid, styrenesulfonic acid, 4-sulfobutyl (meth)acrylate, methallyloxy-benzenesulfonic acid, allyloxybenzensulfonic acid, 2-acrylamide-2-methylpropanesulfonic acid, 2-sulfoethyl (meth)acrylate and metal salt of those monomers while examples of the vinyl monomer containing carboxyl group are acrylic acid, methacrylic acid, maleic acid, itaconic acid, vinylpropionic acid and metal salt of those monomers.

Further, for a polymer which is able to be subjected to a graft polymerization, it is possible to introduce an acidic group thereinto by a graft polymerization of the aforementioned vinyl monomer having the acidic group.

For a polymer having a functional group which is able to be converted to a carboxyl group by hydrolysis such as nitrile, amide and ester, it is possible to introduce a carboxyl group thereinto by subjecting to a hydrolyzing treatment. Examples of the polymer having a functional group which is able to be converted to a carboxyl group by hydrolysis are a monomer having nitrile group such as acrylonitrile and methacrylonitrile; ester derivative such as methyl (meth)acrylate and ethyl (meth)acrylate; amide derivative such as (meth)acrylamide and dimethyl(meth)acrylamide; and polymer of a vinyl type prepared by homopolymerization or copolymerization of anhydride, etc. of a monomer having a carboxyl group such as (meth)acrylic acid, maleic acid and itaconic acid.

The polymer which is prepared by various methods as mentioned above is able to be used as a polymer containing acidic group concerning the present invention and, in view of the fact that many carboxyl groups are able to be made to contain and that many amino acid derivatives are able to be subjected to ionic bond, a polymer of an acrylic acid type is preferred as the polymer containing acidic group and that having a cross-linked structure which will be mentioned later is particularly preferred. Examples of the polymer of an acrylic acid type are water-absorptive resin of an acrylic acid type, water-absorptive fiber such as Belloasis (registered trade mark) manufactured by Kanebo Synthetic Fiber and LANSEAL (registered trade mark) manufactured by Toyobo and hygroscopic fiber such as SUnBUrner manufactured by Toho Textile and MOIS FINE (registered trade mark) manufactured by Toyobo. Incidentally, polymer of an acrylic acid type is a polymer in which most of the acidic group is carboxyl group which is directly bonded to the main chain as represented by polyacrylic acid regardless of the method for manufacturing the same.

With regard to the amount of the acidic group in the polymer containing acidic group, there is a correlation that, when its amount is more, the aforementioned saturated hygroscopic degree and the amount of the amino acid derivative which is able to be bonded are higher. Therefore, an increase in the amount of the acidic group contributes in improvement of the skincare effect but, on the other hand, hydrophilicity of the polymer containing acidic group becomes high whereby there may happen a phenomenon such as that swelling with water becomes vigorous, strength lowers and shape is

crumbled and, in some cases, the polymer *per se* is eluted into water. Degree of the effect and phenomenon as such are also affected by the type of the acidic group. Accordingly, it is preferred that the amount of the acidic group in the polymer containing acidic group is decided by taking the aforementioned matters into consideration. Usually, in the case of carboxyl group, the amount to the weight of the polymer containing acidic group is preferably 1 to 10 mmol/g or, more preferably, 3 to 8 mmol/g.

According to the present invention, the polymer containing acidic group concerning the present invention has a cross-linked structure. Having a cross-linked structure, it suppresses the aforementioned phenomenon of "crumbling of the shape" and "elution of the polymer *per se* into water" and is able to increase the amount of the acidic group. Therefore, it is now possible that much more amino acid derivative is able to be subjected to ionic bond and that far better skincare effect is achieved. There is no particular limitation for such a cross-linked structure and its examples are a cross-linked structure which is formed in such a manner that a monomer having a reactive functional group is copolymerized and is made to react with a compound having plural functions which react with said reactive functional group (hereinafter, referred to as a cross-linking compound) and a cross-linked structure which is formed by copolymerization of a monomer having plural polymerizable functional groups.

In the aforementioned cross-linked structure, there is no particular limitation for a combination of the monomer having a reactive functional group with a cross-linking compound and, in the case of a polymer of a vinyl type, its examples are a combination of a monomer containing nitrile group such as acrylonitrile and methacrylonitrile with a compound of a hydrazine type such as hydrazine hydrate and hydrazine sulfate; a combination of a monomer containing carboxyl group such as acrylic acid and methacrylic acid with a compound containing plural hydroxyl groups such as ethylene glycol and propylene glycol; and a combination of a monomer containing epoxy group such as glycidyl methacrylate with a compound containing plural amino groups such as ethylenediamine and diethylenetriamine.

With regard to the monomer having plural polymerizable functional groups in the latter cross-linking structure, there is no particular limitation and, in the case of a polymer of a vinyl type, a monomer having two or more vinyl groups (hereinafter, may also be referred to as "cross-linking vinyl monomer") corresponds to that. Examples of the cross-linking vinyl monomer as such are triallyl isocyanurate, triallyl cyanurate, divinylbenzene, ethylene glycol di(meth)acrylate, polyethylene glycol di(meth)acrylate, 1,4-butanediol di(meth)acrylate, 1,3-butanediol di(meth)acrylate, 1,6-hexanediol (meth)acrylate, 1,9-nonanediol di(meth)acrylate, trimethylolpropane tri(meth)acrylate, pentaerythritol tetra(meth)acrylate, dipentaerythritol hexa(meth)acrylate, glycerol dimethacrylate, 2-hydroxy-3-acryloyloxypropyl (meth)acrylate, di (meth)acrylate of an adduct of bisphenol A with ethylene oxide, di(meth)acrylate of an adduct of bisphenol A with propylene oxide, neopentyl glycol di(meth)acrylate, 1,10-decanediol di(meth)acrylate, dimethyloltricyclodecane di(meth) acrylate, ethylene oxide-modefied trimethylolpropane tri(meth)acrylate and methylenebisacrylamide.

In the selection of a method for the introduction of carboxyl group under the aforementioned hydrolyzing treatment, it is desirable that the cross-linking is not cleaved even under the hydrolyzing treatment condition. Examples of the cross-linked structure as such are a cross-linked structure formed by the reaction of nitrile group with a compound of a hydrazine type and a cross-linked structure formed by copolymerization of triallyl isocyanurate, triallyl cyanurate, divinyl-bezazene, methylene bisacrylamide, etc.

With regard to a monomer constituting the polymer containing acidic group other than the aforementioned monomer concerning introduction of acidic group and cross-linked structure or, in other words, copolymerizing component, there is no particular limitation but an appropriate selection by taking characteristic, etc. into consideration may be done.

With regard to the shape of the polymer containing acidic group concerning the present invention, there is no particular limitation but, since the shape thereof is able to be a shape of the sustained-release polymer for amino acid derivative according to the present invention as it is, it is usually selected by taking the use of the sustained-release polymer for amino acid derivative into consideration. With regard to the representative shape, its examples are particles and fibrous form and the particles are able to be used for cosmetics, etc. while the fibrous form is able to be used for clothing, etc.

With regard to a method for the manufacture of a polymer containing acidic group, there is no particular limitation but the product is able to be prepared in such a manner that, after polymerization, the aforementioned cross-linking treatment or hydrolyzing treatment is carried out if necessary. There is no particular limitation for the polymerizing method and any of commonly adopted ones such as suspension polymerization, emulsion polymerization, precipitation polymerization, dispersion polymerization and bulk polymerization may be adopted. In the manufacture of a fibrous polymer containing acidic group, it is difficult to make into fiber after cross-linking treatment or hydrolyzing treatment and, therefore, it is common that a spinning step such as melt spinning or wet spinning is conducted after polymerization and the resulting fiber is subjected to a cross-linking treatment or hydrolyzing treatment.

With regard to an advantageous example of fiber-shaped polymer containing acidic group, there may be listed a moisture-absorptive and desorptive fiber of an acrylic acid type prepared by subjecting acrylonitrile fiber to a cross-link introducing treatment by a hydrazine-type compound and to a hydrolyzing treatment by an alkaline metal salt. Even in said fiber, it goes without saying that the aforementioned synergistic effect is achieved and an excellent skincare effect

is able to be achieved if there is a saturated hygroscopic degree of not less than 20 % by weight is available under the condition of 20 °C × 65 % RH. However, since said fiber has moisture-absorptive heat-generating property and antibacterial activity in addition to moisture-absorptive and desorptive property, the fiber is able to be made into more useful sustained-release polymer for amino acid derivative. Said fiber will be illustrated in detail as follows.

With regard to the acrylonitrile fiber which is used as a material fiber for the aforementioned moisture-absorptive and desorptive fiber of an acrylic acid type, it is possible to adopt a fiber formed by an acrylonitrile (hereinafter, may be referred to as AN) polymer containing not less than 40 % by weight, preferably not less than 50 % by weight or, more preferably, not less than 80 % by weight of AN. The AN polymer may be any of homopolymer of AN and copolymer of AN with other monomer and, with regard to a copolymerizing component other than AN, there is no particular limitation so far as it is a monomer which is able to be copolymerized with AN such as a monomer containing sulfonic acid group such as methallylsulfonic acid and p-styrenesulfonic acid and a salt thereof; and a monomer such as styrene, vinyl acetate and methyl (meth)acrylate.

Cross link is formed in an acrylonitrile fiber in such a meaning that a cross-link introducing treatment is conducted by a compound of a hydrazine type and it is no longer dissolved by a solvent for said acrylonitrile fiber and, at the same time, an increase in nitrogen amount takes place as a result thereof wherein there is no particular limitation for the means therefore. A means by which an increase in nitrogen amount by that treatment is able to be adjusted to 1.0 to 10 % by weight is preferred but a means where an increase in nitrogen amount is 0.1 to 1.0 % by weight is able to be adopted as well so far as it is a means by which a polymer containing acidic group which fulfils the characteristic of the sustained-release polymer for amino acid derivative according to the present invention is able to be prepared. With regard to a means which is able to adjust an increase in nitrogen amount to 1.0 to 10 % by weight, a means where treatment is conducted at 50 to 120 °C within 5 hours in an aqueous solution of 5 to 60 % weight concentration of a hydrazine-type compound is industrially preferred. In order to suppress the increase in nitrogen amount low, the above condition is to be made milder according to the teaching of reaction technology. Here, an increase in nitrogen amount is a difference between the nitrogen amount of the material acrylonitrile fiber and the nitrogen amount of the cross-linked acrylonitrile fiber into which a compound of a hydrazine type is introduced.

There is no particular limitation for the compound of a hydrazine type used here and its examples are hydrazine hydrate, hydrazine sulfate, hydrazine hydrochloride, hydrazine hydrobromide and hydrazine carbonate as well as a compound having plural amino groups such as ethylenediamine, guanidine sulfate, guanidine hydrochloride, guanidine phosphate and melamine.

Such a fiber where a cross-link introducing treatment by a hydrazine-type compound is conducted may be subjected to a treatment with an acid after the hydrazine-type compound remained therein by said treatment is fully removed. Examples of the acid used here are mineral acid such as nitric acid, sulfuric acid and hydrochloric acid and organic acid although there is no particular limitation therefore. With regard to the condition for said treatment with an acid, there is no particular limitation and an example thereof is that the fiber to be treated is dipped at 50 to 120 °C for 0.5 to 10 hour(s) in an aqueous solution containing 3 to 20 % by weight or, preferably, 7 to 15 % by weight of an acid.

The fiber which is subjected to a cross-link introducing treatment by a hydrazine-type compound or which is further subjected to a treatment with an acid is then hydrolyzed with an aqueous solution of alkaline metal salt. As a result of such a treatment, hydrolysis of CN group remaining there without participating in the cross-link introducing treatment by a treatment with a hydrazine-type compound of the acrylonitrile fiber or CN group remaining there in case a treatment with an acid is done after the cross-link introducing treatment and $CONH_2$ group produced by hydrolysis of a partial treatment with an acid is carried out. Those functional groups form carboxyl group by hydrolysis and, since the used chemical is an alkaline metal salt, the counter ion is a metal ion. Examples of the alkaline metal salt used here are alkali metal hydroxide, alkali earth metal hydroxide and alkali metal carbonate and examples of the metal species are alkali metal such as Li, Na and K and alkali earth metal such as Mg, Ca and Ba.

Although there is no particular limitation for the condition for a treatment with alkaline metal salt, a means where treatment is conducted in an aqueous solution of 0.5 to 10 % by weight or, preferably, 1 to 5 % by weight at 50 to 120 °C for 1 to 10 hour(s) is preferred in view of industry and properties of the fiber. With regard to the degree of hydrolysis or, in other words, produced amount of carboxyl group, a favorable result is apt to be achieved when it is 1 to 10 mmol/g, preferably, 3 to 10 mmol/g or, more preferably, 3 to 8 mmol/g and that is able to be easily adjusted by combination of concentration of the chemicals in the aforementioned treatment, temperature and treating time. When the amount of carboxyl group is less than 1 mmol/g, a sufficient hygroscopic property may not be available while, when it is more than 10 mmol/g, practically satisfactory properties of the fiber may not be available. Incidentally, CN group may remain or may not remain in the fiber subjected to such a hydrolyzing step. When CN group remains, there is a possibility to give further function utilizing the reactivity thereof.

Fiber which was hydrolyzed may be subjected to a treatment for adjusting the counterion for carboxyl group using a metal salt if necessary. Metal species of the metal salt adopted for such a counterion adjusting treatment is selected from Li, Na, K, Ca, Mg, Ba and A1 and, since the cases of Na, K, Ca and Mg give better moisture absorptive and desorptive property, they are particularly recommended. With regard to the type of the salt used for the treatment,

a water-soluble salt of such a metal will do and its examples are hydroxide, halide, nitrate, sulfate and carbonate. To be more specific, as representative ones for each metal, NaOH and $Na_2CO_3$ for Na salt, KOH for K salt and $Ca(OH)_2$, $Ca(NO_3)_2$ and $CaCl_2$ for Ca salt are appropriate.

With regard to the moisture absorptive and desorptive fiber of an acrylic acid type which is subjected to a cross-link introducing treatment with a hydrazine compound and a hydrolyzing treatment with an alkaline metal salt being adopted in the present invention, that which is subjected to a treatment other than the aforementioned cross-link introducing treatment, treatment with acid, hydrolyzing treatment and counterion adjusting treatment may also be used provided that it does not inhibit the property of the sustained-release polymer for amino acid derivative according to the present invention.

Although the aforementioned moisture absorptive and desorptive fiber of an acrylic acid type is suitable as a fibrous polymer containing acidic group in the present invention, moisture absorptive and desorptive fine particles of an acrylic acid type which are prepared by the same manufacturing steps using acrylonitrile polymer fine particles as a material instead of the acrylonitrile fiber which is a material fiber of said fiber are able to be preferably used as polymer containing acidic group in particles in the present invention. Unlike the case of fibrous form, there is no limitation for fiber property in the case of particles and, therefore, it is possible to adopt the range of 0.1 to 15 % by weight as an increase in nitrogen content and of not less than 1.0 mmol/g as an amount of carboxyl group. Although there is no particular limitation for the upper limit of carboxyl group, it is to be an amount where the skeleton part of the polymer is deduced, the upper limit is 32 mmol/g. Conditions for cross-linking introduction by a hydrazine compound and for hydrolyzing treatment may be appropriately set so as to satisfy those ranges.

Other appropriate examples of the polymer containing acidic group in particles are fine particles prepared in such a manner that a copolymerizable monomer composition containing not less than 50 % by weight of acrylonitrile and further containing divinylbenzene or triallyl isocyanurate and other copolymerable monomer is copolymerized to introduce a cross-linking structure and then nitrile group of the resulting cross-linking acrylonitrile polymer is hydrolyzed to change to carboxyl group and fine particles prepared in such a manner that a copolymerizable monomer composition containing not less than 5 % by weight of methyl acrylate and further containing divinylbenzene or triallyl isocyanurate and other copolymerizable monomer is copolymerized to introduce a cross-linking structure and then a methyl ester moiety of the resulting cross-linking polymer of a methyl acrylate type is hydrolyzed to change to carboxyl group. With regard to polymer containing acidic group in particles as such, it is also possible that, as an amount of carboxyl group, a range of not less than 1.0 mmol/g is adopted.

Then a method for the manufacture of the sustained-release polymer for amino acid derivative according to the present invention will be illustrated. The sustained-release polymer for amino acid derivative according to the present invention is able to be prepared by applying an amino acid derivative solution to the aforementioned polymer containing acidic group followed by drying. There is no particular limitation for the method of applying and a method such as spraying, dipping or painting may be adopted.

With regard to the amino acid derivative solution, it is preferred to be an aqueous solution in view of consideration in environmental load, etc. Even when solubility of the amino acid derivative in water is low, there are many cases where preparation of an aqueous solution is possible when hydrochloride or the like of said amino acid derivative is used. There is no particular limitation for concentration of the amino acid derivative solution and it may be appropriately set so that necessary amount of the amino acid derivative is subjected to ionic bond although it is usually preferred to be 0.5 to 5.0 % by weight.

Although there is no particular limitation for the temperature when the amino acid derivative solution is applied, it is preferably 10 to 80 °C, more preferably 10 to 50 °C and, still more preferably, 20 to 35 °C. Although there is also no particular limitation for the drying temperature, it is preferably 40 to 100 °C, preferably 40 to 80 °C and, still more preferably, 50 to 70 °C. Usually an amino acid derivative is apt to be denatured by heat and, therefore, it is not preferred that the aforementioned applying and drying are conducted at high temperature.

Now, the use of the sustained-release polymer for amino acid derivative according to the present invention will be illustrated. The sustained-release polymer for amino acid derivative according to the present invention is able to be utilized for all uses where a skincare effect is demanded and representative ones thereof are cosmetic and fiber structure.

Firstly, a cosmetic which is characterized in containing said polymer will be illustrated. The cosmetic of the present invention may be in any of the forms of powder, liquid, cream, paste, cake, solid, etc. and specific examples thereof are makeup cosmetic such as foundation, white makeup powder, cheek rouge and eye shadow, body cosmetic such as body powder and baby powder, skincare preparations such as cosmetic lotion, milky lotion, beauty lotion, skin cream and sun care, cleansing agent such as face washing powder, hair shampoo, body shampoo, hand soap and solid soap, hair cosmetic such as rinse, treatment, styling agent, antiperspirant, bathing agent and hairdye, etc.

Compounding amount of the polymer to the cosmetic greatly varies depending upon the form of the cosmetic and, in the case of makeup cosmetic, body cosmetic and skincare preparation, it is 0.01 to 95 % by weight while, in the case of cleansing agent and hair cosmetic, it is 0.01 to 30 % by weight.

There is no particular limitation for other components which are able to be compounded with the cosmetic of

the present invention and various components which have been commonly used as cosmetic materials may be used depending upon the form of each cosmetic used. For example, various components such as inorganic powder, organic powder, dispersing agent, oil agent, emulsifier, surfactant, thickener, antiseptic, fragrance, ultraviolet absorber (including that of organic and inorganic types; may correspond to any of UV-A and B), moisturizer, physiologically active component, salt, solvent, antioxidant, antibacterial, antiperspirant, chelating agent, neutralizing agent and pH adjusting agent may be compounded at the same time.

Now, the fiber structure which is characterized in containing the sustained-release polymer for amino acid derivative according to the present invention will be illustrated. In the case of the fiber structure, the polymer is able to be contained therein by fixing the sustained-release polymer for amino acid derivative in particles to the fiber surface constituting the fiber structure or by using the sustained-release polymer for amino acid derivative in fibrous shape as a constituting fiber.

There is no particular limitation for other material which is able to be used jointly in the fiber structure containing the sustained-release polymer for amino acid derivative according to the present invention and commonly used natural fiber, organic fiber, semi-synthetic fiber and synthetic fiber may be used. Inorganic fiber, glass fiber, etc. may also be used depending upon the use. Specific examples are cotton, linen, silk, wool, nylon, rayon, polyester and acrylic fiber.

When the sustained-release polymer for amino acid derivative in fibrous shape is used together with other fiber to give a fiber structure, the using amount of the sustained-release polymer for amino acid derivative in a fibrous form is preferably 3 % by weight to less than 100 % by weight or, more preferably, 5 % by weigh to 50 % by weight.

The aforementioned fiber structure containing the sustained-release polymer for amino acid derivative according to the present invention has an excellent skincare effect and has a good hand. Especially when the fiber structure has a saturated hygroscopic degree of not less than 5 % by weight or, preferably, not less than 8 % by weight under the condition of 20°C and 65 % RH, more excellent skincare effect is able to be achieved.

Examples of the fiber structure containing the sustained-release polymer for amino acid derivative according to the present invention are thread, yarn (including wrap yarn), filament, woven thing, knitted thing, nonwoven fabric, paper-shaped thing, sheet-shaped thing, layered product and cotton-shaped thing (including that in spheres and in blocks) and, in view of being utilized to clothing etc. contacting to the skin, woven thing and knitted thing are most common. Examples of specific shape thereof are underwear, stomach band, supporter, mask, gloves, socks, stockings, pajama, bathrobe, towel, mat and bedclothes.

The fiber structure containing the sustained-release polymer for amino acid derivative according to the present invention is able to be manufactured by using fiber to which the sustained-release polymer for amino acid derivative in particles is fixed or the sustained-release polymer for amino acid derivative in a fibrous form together, in some cases, with other material by mixed spinning, or it is able to be manufactured by fixing of the sustained-release polymer for amino acid derivative in particles to a previously-prepared fiber structure. There is no particular limitation for a method of fixing the sustained-release polymer for amino acid derivative in particles, and a binder or the like may be used therefore.

Besides the aforementioned methods, the fiber structure containing the sustained-release polymer for amino acid derivative according to the present invention may also be manufactured by applying an amino acid derivative solution to the fiber structure containing polymer containing acidic group followed by drying. With regard to applying and drying in that case, the same method and condition as in the case of the aforementioned method for the manufacture of the sustained-release polymer for amino acid derivative may be adopted.

A big characteristic feature of the aforementioned sustained-release polymer for amino acid derivative according to the present invention is that an amino acid derivative is subjected to ionic bond to acidic group of the polymer containing acidic group. Since ionic bond is a reversible bond, when the sustained-release polymer for amino acid derivative according to the present invention contacts to moisture or, particularly, to water containing electrolytic salt such as sweat, the amino acid derivative is gradually released. Due to the action as such, a good skincare effect is achieved and, conversely, a bonding amount of the amino acid derivative in the sustained-release polymer for amino acid derivative decreases upon use. When the bonding amount of the amino acid derivative decreases, it is a matter of course that the skincare effect also lowers but the sustained-release polymer for amino acid derivative according to the present invention is able to be regenerated by using its characteristic that an amino acid derivative can be contained due to its ionic bond.

In a method for the regenerating treatment, an amino acid derivative solution is applied to the sustained-release polymer for amino acid derivative in which bonding amount of the amino acid derivative lowered upon use or to the fiber structure containing said polymer and then dried. As a result of such a treatment, the amino acid derivative is subjected to ionic bond again and a skincare effect is able to be expressed. In the case of a fiber structure containing the sustained-release polymer for amino acid derivative such as underwear, the regenerating treatment is able to be carried out in such a manner that, after finishing the rinsing upon washing at home, the amino acid derivative is added to the tub of a washing machine either directly or after mixing with a softeners or that the amino acid derivative solution is sprayed after dehydration and that is very practical.

## Examples

The present invention will now be specifically illustrated by way of the following Examples but they are mere examples and the gist of the present invention is not limited by them. Incidentally, the terms "part(s)" and "percentage" in the Examples are those by weight unless otherwise mentioned. Method for the manufacture of the polymer containing acidic group used in the Examples is as follows.

[Moisture absorptive and desorptive fiber A of an acrylic acid type]

An original liquid for spinning in which 10 parts of AN polymer (limiting viscosity at 30 °C in dimetliylformamide [η]: 1.2) comprising 90 % of AN and 10 % of vinyl acetate was dissolved in 90 parts of 48 % aqueous solution of sodium thiocyanate was subjected to spinning and elongation by a conventional method (total elongation rate: 10-fold) and subjected to drying and moist heating treatment in an atmosphere where (dry bulb)/(wet bulb) = 120 °C/60 °C to give a material fiber (a) where single fiber fineness was 0.9 dtex. The material fiber (a) was subjected to a treatment for introduction of cross-linking at 98 °C for 5 hours in a 20 % aqueous solution of hydrazine hydrate and washed with water. After that, it was subjected to a treatment with acid at 90 °C for 2 hours in a 3 % aqueous solution of nitric acid. Then, it was subjected to a hydrolyzing treatment at 90 °C for 2 hours in a 1 % aqueous solution of sodium hydroxide, adjusted to pH 12 and washed with pure water to give moisture absorptive and desorptive fiber A of an acrylic acid type, In the fiber, acidic group was 1.2 mmol/g and saturated hydroscopic degree was 18 %.

[Moisture absorptive and desorptive fiber B of an acrylic acid type]

The same method as in the case of the moisture absorptive and desorptive fiber A of an acrylic acid type was conducted except that the concentration of sodium hydroxide in a hydrolyzing treatment of the material fiber (a) was made 3 % to give a moisture absorptive and desorptive fiber B of an acrylic acid type. In the fiber, acidic group was 6.1 mmol/g and saturated hydroscopic degree was 57 %.

[Moisture absorptive and desorptive fiber C of an acrylic acid type]

The same method as in the case of the moisture absorptive and desorptive fiber A of an acrylic acid type was conducted except that the concentration of sodium hydroxide in a hydrolyzing treatment of the material fiber (a) was made 10 % to give a moisture absorptive and desorptive fiber C of an acrylic acid type. In the fiber, acidic group was 8.8 mmol/g and saturated hydroscopic degree was 75 %.

[Acrylic fiber D containing itaconic acid]

The same method as in the case of the moisture absorptive and desorptive fiber A of an acrylic acid type was conducted except that an acrylonitrile polymer (limiting viscosity at 30 °C in dimethylformamide [η]: 1.2) comprising 94 % of acrylonitrile and 6 % of itaconic acid was used in the preparation of the material fiber (a) to give an acrylic fiber D containing itaconic acid In the fiber, acidic group was 0.9 mmol/g and saturated hydroscopic degree was 7 %.

[Particles E containing acidic group]

A water-soluble polymer (300 parts) in which methacrylic acid/sodium p-styrenesulfonate = 70/30 and 30 parts of sodium sulfate were dissolved in 6,595 parts of water and placed in a polymerization tank equipped with an oar-shaped stirrer. Then 15 parts of 2, 2'- azobis(2,4-dirnethylvaleronitrile) was dissolved in 2,700 parts of acrylonitrile and 300 parts of divinylbenzene, placed in a polymerization tank and subjected to a suspension polymerization at 60 °C for 2 hours under a stirring condition of 400 rpm to give material particles of 87 % polymerization degree. The material particles (100 parts) were dispersed in 900 parts of deionized water, 100 parts of sodium hydroxide was added thereto, the mixture was subjected to a hydrolyzing reaction at 90 °C for 2 hours, pH was adjusted to 12 and the resulting particles were washed, dehydrated, dried and pulverized to give particles E containing acidic group. In the particles, average particle size was 10 μm, amount of the acidic group was 5.5 mmol/g and saturated hydroscopic degree was 45 %.

[Particles F containing acidic group]

A water-soluble polymer (300 parts) in which methacrylic acid/sodium p-styrenesulfonate = 70/30 and 30 parts of sodium sulfate were dissolved in 6,595 parts of water and placed in a polymerization tank equipped with an oar-shaped stirrer. Then 15 parts of 2, 2'- azobis(2,4-dimethylvaleroutitile) was dissolved in 2,700 parts of acrylonitrile and

300 parts of methyl acrylate, placed in a polymerization tank and subjected to polymerization at 60 °C for 2 hours under a stirring condition of 400 rpm to give material particles of 87 % polymerization degree. The material particles (100 parts) were mixed with 100 parts of 60 % by weight of hydrazine and 800 parts of water, heated at 90 °C for 3 hours to introduce cross-linking thereinto, then 100 parts of sodium hydroxide was added thereto, the mixture was subjected to a hydrolyzing reaction at 120 °C for 2 hours, pH was adjusted to 12 and the resulting particles were washed, dehydrated, dried and pulverized to give particles F containing acidic group. In the particles, average particle size was 12 μm, amount of the acidic group was 3.4 mmol/g and saturated hydroscopic degree was 38 %.

[Example 1]

Arginine hydrochloride (manufactured by Tokyo Kasei Kogyo K. K.) was dissolved in a solution comprising methanol/water in a ratio of 7/93 to prepare a 1.0 % aqueous arginine hydrochloride solution. LANSEAL F manufactured by Toyobo K. K. was dipped in the solution in a bath ratio of 1/20 at 25 °C for 30 minutes, washed with running water for 5 minutes and dried with a hot-air drier of 70 °C to give a sustained-release polymer for amino acid derivative in a fibrous shape.

[Example 2]

Arginine hydrochloride (manufactured by Tokyo Kasei Kogyo K. K.) was dissolved in water to prepare a 1.0 % aqueous arginine hydrochloride solution. The aforementioned moisture absorptive and desorptive fiber A of an acrylic acid type was dipped in the solution in a bath ratio of 1/20 at 25 °C for 30 minutes, washed with running water for 5 minutes and dried with a hot-air drier of 70 °C to give a sustained-release polymer for amino acid derivative in a fibrous shape.

[Example 3]

Arginine hydrochloride (manufactured by Tokyo Kasei Kogyo K. K.) was dissolved in water to prepare a 3.0 % aqueous arginine hydrochloride solution. The aforementioned moisture absorptive and desorptive fiber B of an acrylic acid type was dipped in the solution in a bath ratio of 1/20 at 25°C for 30 minutes, washed with running water for 5 minutes and dried with a hot-air drier of 70°C to give a sustained-release polymer for amino acid derivative in a fibrous shape.

[Example 4]

Arginine hydrochloride (manufactured by Tokyo Kasei Kogyo K. K.) was dissolved in water to prepare a 5.0 % aqueous arginine hydrochloride solution. The aforementioned moisture absorptive and desorptive fiber C of an acrylic acid type was dipped in the solution in a bath ratio of 1/20 at 25 °C for 30 minutes, washed with running water for 5 minutes and dried with a hot-air drier of 70 °C to give a sustained-release polymer for amino acid derivative in a fibrous shape.

[Example 5]

Histidine hydrochloride (manufactured by Tokyo Kasei Kogyo K. K.) was dissolved in water to prepare a 3.0 % aqueous histidine hydrochloride solution. The aforementioned moisture absorptive and desorptive fiber B of an acrylic acid type was dipped in the solution in a bath ratio of 1/20 at 25 °C for 30 minutes, washed with running water for 5 minutes and dried with a hot-air drier of 70 °C to give a sustained-release polymer for amino acid derivative in a fibrous shape.

[Example 6]

Lysine hydrochloride (manufactured by Tokyo Kasei Kogyo K. K.) was dissolved in water to prepare a 3.0 % aqueous lysine hydrochloride solution. The aforementioned moisture absorptive and desorptive fiber B of an acrylic acid type was dipped in the solution in a bath ratio of 1/20 at 25 °C for 30 minutes, washed with running water for 5 minutes and dried with a hot-air drier of 70°C to give a sustained-release polymer for amino acid derivative in a fibrous shape.

[Example 7]

Sericin A-30F (manufactured by K. K. Shinko Silk) was dissolved in water and converted to a form of a hydrochloride to prepare a 5.0 % aqueous sericin solution. The moisture absorptive and desorptive fiber B of an acrylic acid type was dipped in the solution in a bath ratio of 1/20 at 25 °C for 30 minutes, washed with running water for 5 minutes and dried with a hot-air drier of 70°C to give a sustained-release polymer for amino acid derivative in a fibrous shape.

[Example 8]

Arginine hydrochloride (manufactured by Tokyo Kasei Kogyo K. K.) was dissolved in water to prepare a 1.0 % aqueous arginine hydrochloride solution. The aforementioned acrylic fiber D containing itaconic acid was dipped in the solution in a bath ratio of 1/20 at 25 °C for 30 minutes, washed with running water for 5 minutes and dried with a hot-air drier of 70 °C to give a sustained-release polymer for amino acid derivative in a fibrous shape.

[Comparative Example 1]

Arginine hydrochloride (manufactured by Tokyo Kasei Kogyo K. K.) was dissolved in water to prepare a 5.0 % aqueous arginine hydrochloride solution. Polyester fiber 2T38 manufactured by Toyobo was dipped in the solution in a bath ratio of 1/20 at 25°C for 30 minutes, washed with running water for 5 minutes and dried with a hot-air drier of 70 °C to give a fibrous polymer.

[Example 9]

Circular rib knitted cloth prepared from spun yarn constituted from 30 % of moisture absorptive and desorptive fiber B of an acrylic acid type, 30 % of rayon and 40 % of polyester was dipped in a 0.3 % aqueous solution of arginine hydrochloride (manufactured by Ajinomoto) in a bath ratio of 1/50 at 40 °C for 30 minutes, washed with running water for 5 minutes and dried with a hot-air drier of 70 °C to give a knitted cloth containing fibrous sustained-release polymer for amino acid derivative.

[Example 10]

The same operation as in Example 9 was conducted except that a 10 % aqueous solution of arginine hydrochloride (manufactured by Ajinomoto) was used to give a knitted cloth containing fibrous sustained-release polymer for amino acid derivative.

[Example 11]

Particles E containing acidic group were dipped in a 0.3 % aqueous solution of arginine hydrochloride (manufactured by Ajinomoto) in a bath ratio of 1/50 at 25 °C for 30 minutes, washed with water and dehydrated using a centrifugal dehydrating machine and dried at 30 °C to give a sustained-release polymer for amino acid derivative in particles.

[Example 12]

Particles F containing acidic group were dipped in a 0.3 % aqueous solution of arginine hydrochloride (manufactured by Ajinomoto) in a bath ratio of 1/50 at 25 °C for 30 minutes, washed with water and dehydrated using a centrifugal dehydrating machine and dried at 30 °C to give a sustained-release polymer for amino acid derivative in particles.

[Test Example 1]

The products of Examples 1 to 8 and Comparative Examples 1 and 2 were evaluated for amount of acidic group, saturated hygroscopic degree, initial binding amount of the amino acid derivative, hand and effect to the skin by the following methods. Result of the evaluation is shown in Table 1.

<Method for measurement of amount of acidic group (amount of carboxyl group)>

Well-dried polymer containing acidic group (about 1 g) was precisely weighted (X gram(s)), 200 ml of water was added thereto, 1 ml/L aqueous solution of hydrochloric acid was added thereto with warming to 50 °C to make pH 2 and a titration curve was determined according to a conventional method using a 0.1 mol/L aqueous solution of sodium hydroxide. From the titration curve, amount of aqueous solution of sodium hydroxide (Y ml) consumed for the acidic

group was determined and amount of carboxyl group (mmol/g) was calculated from the following formula.

$$\text{Amount of carboxyl group (mmol/g)} = 0.1Y/X$$

<Method for measurement of saturated hygroscopic degree>

A sample (about 5.0 g) was dried at 105 °C for 16 hours using a hot-air drying machine to measure its weight (W1 gram(s)). Then the sample was placed in a constant-humidity tank of 20 °C and 65 % RH for 24 hours. Weight of the sample which absorbed moisture as such was measured (W2 gram(s)). From the above measured result, calculation was conducted by the following formula.

$$\text{(Saturated hygroscopic degree) (\%)} = \{(W2 - W1)/W1\} \times 100$$

<Method for measurement of initial binding amount of amino acid derivative>

With regard to the initial binding amount of amino acid derivative, a sample (0.5 g) of sustained-release polymer for amino acid derivative was dipped in 25 mL of 0.5N aqueous solution of hydrochloric acid at 40 °C for 30 minutes to extract the amino acid derivative. After that, when the sample was fiber or knitted cloth, the sample was squeezed or, when the sample was particles, it was filtered whereupon an extract was prepared. The resulting extract was subjected to a quantitative analysis using an analytical method which is suitable for each of the compounds and amount of the extracted amino acid derivative was determined. When the amino acid derivative was a basic amino acid, a quantitative analysis was conducted using an amino acid analyzer (L-8800 manufactured by HITACHI). From a change in weight when dried at 40 °C for 12 hours *in vacuo* in a desiccator in which diphosphorus pentaoxide was placed, moisture amount of the sample was determined. From the amount of the extracted amino acid derivative and moisture amount of the sample, the initial binding amount of the amino acid derivative per gram of the sample of sustained-released polymer for amino acid derivative was determined by calculation.

<Method for evaluation of hand of the sample>

Fiber material was spun by a conventional method to prepare a spun yarn having 40/1 count cotton yarn, the spun yarn was then subjected to a rib stitch in 16 gages and 2 plies to give knitted cloth where unit weight was about 200 g/$m^2$ and the knitted cloth was evaluated in terms of the feel upon grasping by the following method.
oo: very soft hand
o: soft hand
Δ: a little soft hand
×: no soft feel but hard hand

<Method for evaluation of the effect to the skin>

The knitted cloth prepared as above was applied to the elbow of five persons to be tested who were in dry skin and the state of the skin at the elbow after seven days from the application was evaluated according to the following five ranks to calculate the mean value.
+2: particularly moist
+1: moist
0: normal
-1: rough skin
-2: particularly rough skin
[Table 1]

| | Polymer Containing Acidic Group | Amount of Acidic Group (mmol/g) | Saturated Hygroscopic Degree (%) | Amino Acid Derivative | Initial Binding Amount of Amino Acid Derivative | Method for Application | Hand | Effect to the Skin |
|---|---|---|---|---|---|---|---|---|
| Ex 1 | LANSEAL | 2.0 | 9 | arginine | 8 | ionic bond | Δ | +1.0 |
| Ex 2 | MADF A of Acrylic Acid Type | 1.2 | 18 | arginine | 4 | ionic bond | o | +0.8 |
| Ex 3 | MADF B of Acrylic Acid Type | 6.1 | 57 | arginine | 25 | ionic bond | oo | +1.6 |
| Ex 4 | MADF C of Acrylic Acid Type | 8.8 | 75 | arginine | 37 | ionic bond | oo | +1.6 |
| Ex 5 | MADF B of Acrylic Acid Type | 6.1 | 57 | histidine | 6 | ionic bond | oo | +0.6 |
| Ex 6 | MADF B of Acrylic Acid Type | 6.1 | 57 | lysine | - 13 | ionic bond | oo | +1.2 |
| Ex 7 | MADF B of Acrylic Acid Type | 6.1 | 57 | sericin | 4 | ionic bond | o | +0.8 |
| Ex 8 | MADF D Containing Itaconic Acid | 0.9 | 7 | arginine | 3 | ionic bond | Δ | +0.6 |
| CE 1 | Polyester | none | ≤ detective limit | arginine | 0.4 | dipping only | × | -0.8 |
| CE: Comparative Example<br>MADF: Moisture absorptive and desorptive fiber | | | | | | | | |

As shown in Table 1, the knitted cloth prepared from the sustained-release polymers for amino acid derivative of Examples 1 to 8 had a high effect to the skin and showed a good hand. Especially in Examples 3, 4 and 6, binding amount of the amino acid derivative is high showing an excellent property. That is presumably because the amount of acidic group in the polymer containing acidic group is much and the amino acid derivative has a basic functional group whereby ionic bond is efficiently formed. Incidentally, the content of sericin was calculated from the difference of the fiber weights before and after application of sericin.

On the contrary, in Comparative Example 1, polyester fiber containing no acidic group was used and, therefore, the amino acid derivative was not fixed and the effect was hardly available.

From those results for the evaluation, it was noted that, in the present invention, very high amount of amino acid derivative was able to be bonded.

[Test Example 2]

Eluted amount of amino acid derivative

Result of measurement of eluted amount of amino acid derivative for Examples 10 to 12 and comparative Example 1 by the following method is shown in Table 2.

<Method for measurement of eluted amount of amino acid derivative when dipped in artificial sweat or in pure water>

A sample (0.5 g) of the sustained-release polymer for amino acid derivative was dipped in 25 mL of artificial sweat which is shown below or in pure water at 25 °C for 30 minutes to extract. After that, when the sample was fiber or knitted cloth, the sample was squeezed and, when the sample was particles, it was filtered whereupon an extract was prepared. The resulting extract was subjected to a quantitative analysis using an analytical method which is suitable for each of the compounds and amount of the extracted amino acid derivative was determined. When the amino acid derivative was a basic amino acid, a quantitative analysis was conducted using an amino acid analyzer (L-8800 manufactured by HITACHI). From a change in weight when dried at 40°C for 12 hours *in vacuo* in a desiccator in which diphosphorus pentaoxide was placed, moisture amount of the sample was determined. From the amount of the extracted amino acid derivative and moisture amount of the sample, the eluted amount of the amino acid derivative per gram of the sustained-release polymer sample for amino acid derivative upon dipping in artificial sweat or in pure water was determined by calculation.

With regard to the artificial sweat, the following acidic sweat was prepared by referring to JIS-L-0848 and used. With regard to the liquid composition of the acidic sweat, a product where 5 g/L of NaCl and 2.26 g of sodium dihydrogenphosphate dodecahydrate were dissolved in water, the mixture was adjusted to pH 5.5 using 0.5M aqueous solution of NaOH and its volume was adjusted to 1 L was used. With regard to pure water, pure water where electric resistance was not less than 1 MΩ.cm and TOC was not more than 10 ppm prepared using an apparatus for the manufacture of pure water (manufactured by Nippon Millipore) was used.

Eluting rate ($\alpha$) of the amino acid derivative when dipped in artificial sweat and eluting rate ($\beta$) of the amino acid derivative when dipped in pure water were calculated by the following calculation formulae.
Eluting rate ($\alpha$) of the amino acid derivative when dipped in artificial sweat = [(Eluted amount of the amino acid derivative when dipped in artificial sweat) $\div$ (Initial bonding amount of the amino acid derivative)] $\times$ 100 (%)
Eluting rate ($\beta$) of the amino acid derivative when dipped in pure water = [(Eluted amount of the amino acid derivative when dipped in pure water) + (Initial bonding amount of the amino acid derivative)] $\times$ 100 (%)

[Table 2]

| | Bonded Amount of Amino Acid Deriv. (mg/g) | Eluted Amount (mg/g) | | Eluted Rate (%) | | $\alpha/\beta$ |
|---|---|---|---|---|---|---|
| | | Artificial Sweat | Pure Water | Artificial Sweat ($\alpha$) | Pure Water ($\beta$) | |
| Ex 10 | 161.2 | 134.5 | 4.2 | 83.4 | 2.6 | 32.0 |
| Ex 11 | 168.8 | 99.4 | 9.5 | 58.9 | 5.6 | 10.5 |
| Ex 12 | 110.6 | 64.3 | 7.7 | 58.1 | 7.0 | 8.4 |
| CE 1 | 4.3 | 4.2 | 4.2 | 97.7 | 97.7 | 1.0 |
| CE: Comparative Example | | | | | | |

As shown in Table 2, it is noted that, in Examples 10 to 12 which are examples of the present invention, the products which satisfy the condition for achieving so significant skincare effect that eluting rate for artificial sweat is not less than 10 % and that $\alpha/\beta$ is not less than 5 were prepared. On the contrary, in Comparative Example 1, most of the amino acid derivatives are eluted both for artificial sweat and for pure water. Thus, it is noted that, in order to satisfy the condition by which the aforementioned significant skincare effect is achieved, adoption of ionic bond as a method for retaining the amino acid derivative is effective as in the present invention.

[Test Example 3]

Test for recovery of skin moisture

Recovery rate of skin moisture was measured by the following method for the knitted cloth of Example 9 and for the knitted cloth before arginine was applied to said knitted cloth (hereinafter, referred to as unprocessed cloth). Incidentally, bonded amount of the amino acid derivative in the knitted cloth of Example 9 was 79.4 mg/g.

<Test method for recovery of skin moisture>

On the inner side of forearm of a person to be tested, six measuring areas each being 3 cm × 3 cm were set, the person was placed in a room where temperature and moisture were kept constant at 22 °C/40 % RH and, after 15 minutes in a rest state, skin moisture (A) in normal stage was measured using a skin moisture meter (Corneometer CM 825). The measured value was determined by average of the nine areas within the measuring region.

Then, 1 mL of 10 % aqueous solution of potassium laurate was dropped onto the inside of the forearm where the measuring region was set, washed for 1 minutes lightly as if being rubbed, washed out with warm water of 35°C for 30 seconds and a series of operations as such (one cycle) was conducted for three cycles whereby the measuring region on the inside of the forearm was made into a model dry skin. After that, the person was placed in a chamber kept at constant temperature and moisture of 22°C/40 % RH and, after 15 minutes in a rest state, the skin moisture (B) of the model dry skin was measured by the same method as above.

Then the knitted cloth of Example 9 (for three places) and unprocessed cloth (for three places) were adhered on the six measuring regions of the model dry skin and, after 5 hours in a circumstance of 20 to 24.5 °C temperature and 15 to 23 % RH moisture, the person was placed in a chamber kept at constant temperature and moisture of 22 °C/ 40 % RH. After 15 minutes in a rest state, the skin moisture (C) after the application was measured by the same manner as above.

From the above measured results, decreased amount of skin moisture by preparation of the model dry skin (D = A - B) and increased amount of skin moisture recovered by adhesion of the cloth (R = C - B) were measured for each measuring region. Then, from those values, recovery rate of the skin moisture (X = R/D) was calculated and, for each of the knitted cloth of Example 9 and unprocessed cloth, a mean value of the recovery rate of skin moisture was determined. As shown in Fig. 1, the result was 52.7 % in the amino acid-processed cloth and 24.6 % in the unprocessed cloth and it was shown that, when the knitted cloth of Example 9 was applied, recovery rate of skin moisture became high in around twice as much. From the result, it is noted that a good skincare effect is achieved when the fiber structure containing the sustained-release polymer for amino acid derivative according to the present invention is used for such a use that is contacted to the skin.

[Test Example 4]

Regeneration test

Two sheets of knitted cloth (30 × 60 cm) of Example 9 were dipped in a 0.05 % aqueous solution of a detergent ("Top" manufactured by Lion) at room temperature, stirred for 5 minutes, dehydrated and rinsed. After such a series of operation (one cycle) was conducted for five cycles, the sheets of knitted cloth were dehydrated and dried to give a sample where bonding amount lowered due to washing. Bonding amount of the amino acid derivative in the sample was 29.9 mg/g.

Then the sample was dipped in a 1 % aqueous solution of arginine hydrochloride in a bath ratio of 1/50 at 25 °C for 30 minutes, squeezed, rinsed with 2 L of water, dehydrated and dried at 25 °C. Bonding amount of the amino acid derivative in the resulting knitted cloth was 87.6 mg/g and the amino acid derivative was able to be applied again. That shows the skincare effect of the sustained-release polymer for amino acid derivative according to the present invention is able to be recovered by a regeneration treatment.

[Test Example 5]

Evaluation of bulk powder of sustained-release polymer for amino acid derivative in particles

Each 0.5 g of the sustained-released polymer for amino acid derivative in particles of Example 11 (hereinafter, referred to as amino acid-processed particles) and particles E containing acidic group (hereinafter, referred to as unprocessed particles) was placed on one hand, water was added thereto to dissolve and to be made to fit to the skin, rinsed with water and dried with towel and the feel of the skin after drying was compared. In the case of the amino acid-processed particles, the skin was smoother than in the case of the unprocessed particles.

[Test Example 6]

[Evaluation in face-washing powder of a fatty acid salt type]

In accordance with the component ratios as shown in Table 3, a face-washing powder to which the aforementioned amino acid-processed particles were added (added product) and that to which they were not added (non-added

product) were prepared. Hand was washed using them and dried by towel. Comparison was done for foaming during the washing of the hand and for feel of the skin after drying. In the face-washing powder to which amino acid-processed particles were added, foaming was good, squeaking feel upon rinsing was little and feel of the skin after drying was soft and smooth.

[Table 3]

| Components | Added Product | Non-Added Product |
|---|---|---|
| Sodium laurate LN-1 (manufactured by NOF) | 59.4 % | 60% |
| Potassium myristate MK-1 (manufactured by NOF) | 38.6 % | 40 % |
| Amino acid-processed particles | 1 % | 0% |

[Test Example 7]

Evaluation of face-washing powder of an acylglutamate type

In accordance with the component ratios as shown in Table 4, a face-washing powder to which the aforementioned amino acid-processed particles were added (added product) and that to which they were not added (non-added product) were prepared. Hand was washed using them and dried by towel. Comparison was done for foaming during the washing of the hand and for feel of the skin after drying. In the face-washing powder to which amino acid-processed particles were added, foaming was quick and foam amount was much and feel of the skin after drying was soft and smooth.

[Table 4]

| Components | Added Product | Non-Added Product |
|---|---|---|
| Sodium lauroylglutamate; "Amisoft" LS-11 | 18 % | 18 % |
| Sodium myristoylglutamate; "Amisoft" MS-11 | 12 % | 12 % |
| Talc; Microace | 10% | 10 % |
| Mannitol; Marine Crystal | 20% | 20 % |
| Starch; ST Starch C(W) | 38.3 % | 39.8% |
| Methylparaben | 0.2 % | 0.2 % |
| Amino acid-processed particles | 1.5 % | 0 % |

[Test Example 8]

Evaluation in powder foundation

The aforementioned amino acid-processed particles were added in a ratio of 3 parts to 97 parts of foundation manufactured by Kanebo (Kanebo Revue Natural Stay Pact) to prepare a powder foundation. Appearance, smell and feel upon application were evaluated for the powder foundation and were found to be good as foundation.

[Test Example 9]

Evaluation in lipstick

In accordance with the composition ratio as shown in Table 5, a lipstick to which the aforementioned amino acid-processed particles were added was prepared by a conventional method. The resulting lipstick to which the amino acid-processed particles were added was with good spread and with smooth and moist feel.

[Table 5]

| Components | Amount |
|---|---|
| Hydrogenated polyisobutene [PARLEAM 18 (manufactured by NOF)] | 14 % |
| Octyldodecyl myristate | 23.5 % |
| Glyceryl tri(caprylate/caprate) [Myritol 318 (manufactured by Cognis)] | 5 % |
| Octyldodecanol | 20 % |
| Di(phytosteryl/octyldodecyl) lauroylglutamate [Eldew PS-203 (manufd by Ajinomoto)] | 10 % |
| Ozocerite [Ozocerite 7366 (manufactured by Cognis)] | 7.5 % |
| Carnauba wax | 5 % |
| Candelilla wax | 5 % |
| Pigment | 8% |
| Amino acid-processed particles | 2% |

## Claims

1. Sustained-release polymer for amino acid derivative, wherein a polymer containing acidic group is ionically bonded to an amino acid derivative, wherein the polymer containing acidic group has a cross-linked structure and wherein the eluting rate ($\alpha$) of the amino acid derivative when the polymer is dipped in an artificial sweat liquid is 10 % or more and is five times or more of the eluting rate ($\beta$) of the amino acid derivative when the polymer is dipped in pure water.

2. The sustained-release polymer for amino acid derivative according to claim 1, wherein the polymer is able to be regenerated when a solution of the amino acid derivative is impregnated thereinto after release of the amino acid derivative.

3. The sustained-release polymer for amino acid derivative according to claims 1 or 2, wherein the amino acid derivative has the structure as shown by the following formula [I] in its molecule.

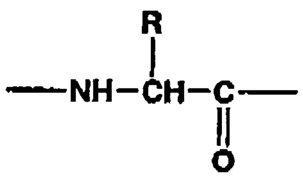

[I]

wherein R is a group having one or more basic functional group(s).

4. The sustained-release polymer for amino acid derivative according to any of claims 1 to 3, wherein the amino acid derivative is a basic amino acid.

5. The sustained-release polymer for amino acid derivative according to any of claims 1 to 4, wherein the amino acid derivative is at least one member selected from the group consisting of arginine, lysine and histidine.

6. The sustained-release polymer for amino acid derivative according to any of claims 1 to 5, wherein the polymer containing acidic group has a saturated hygroscopic degree of 20 % by weight or more under the condition of 20 °C × 65 % RH.

7. The sustained-release polymer for amino acid derivative according to any of claims 1 to 6, wherein the polymer containing acidic group has a carboxyl group.

8. The sustained-release polymer for amino acid derivative according to any of claims 1 to 7, wherein the polymer

containing acidic group is a polymer of an acrylic acid type.

**9.** The sustained-release polymer for amino acid derivative according to any of claims 1 to 8, wherein the cross-linked structure is formed by the reaction of nitrile group with a hydrazine type compound.

**10.** The sustained-release polymer for amino acid derivative according to any of claims 1 to 8, wherein the cross-linked structure is formed by copolymerization of a cross-linking vinyl monomer.

**11.** The sustained-release polymer for amino acid derivative according to any of claims 1 to 10, wherein the polymer containing acidic group is in particles.

**12.** The sustained-release polymer for amino acid derivative according to any of claims 1 to 10, wherein the polymer containing acidic group is fibrous.

**13.** A method for the manufacture of the sustained-release polymer for amino acid derivative mentioned in any of claims 1 to 12, wherein a solution of amino acid derivative is added to a polymer containing acidic group and then the polymer is dried at 40 to 100°C.

**14.** A cosmetic containing the sustained-release polymer for amino acid derivative mentioned in claim 11.

**15.** A fiber structure containing the sustained-release polymer for amino acid derivative mentioned in claim 11 or 12.

**16.** The fiber structure according to claim 15, wherein the fiber structure is selected from underwear, stomach band, supporter, mask, gloves, socks, stockings, pyjama, bathrobe, towel, mat and bedclothes.

**17.** A method for the manufacture of the fiber structure mentioned in claim 15 or 16, wherein a solution of amino acid derivative is added to a material fiber structure which contains a polymer containing acidic group and then the fiber structure is dried at 40 to 100 °C.

**18.** A method for regeneration of a sustained-release polymer for amino acid derivative, wherein a solution of amino acid derivative is added to the sustained-release polymer for amino acid derivative mentioned in any of claims 1 to 12 or to the fiber structure mentioned in claim 15 or 16 in which amount of the amino acid derivative bonded thereto has lowered as a result of use and then the polymer or the fiber structure is dried.

## Patentansprüche

**1.** Retardierungspolymer (Sustained-release-Polymer) für ein Aminosäurederivat, wobei ein saure Gruppen enthaltendes Polymer mit einem Aminosäurederivat ionisch verbunden ist, wobei das saure Gruppen enthaltende Polymer eine vernetzte Struktur aufweist und wobei die Elutionsrate (α) des Aminosäurederivats beim Eintauchen des Polymers in einer Flüssigkeit aus künstlichem Schweiß bei 10 % und mehr liegt und dabei das Fünffache und mehr der Elutionsrate (β) des Aminosäurederivats beim Eintauchen des Polymers in reinem Wasser beträgt.

**2.** Retardierungspolymer für Aminosäurederivat nach Anspruch 1, bei dem man das Polymer wieder regenerieren kann, indem man es nach Abgabe des Aminosäurederivats mit einer Lösung des Aminosäurederivats imprägniert.

**3.** Retardierungspolymer für Aminosäurederivat nach den Ansprüchen 1 oder 2, bei dem das Aminosäurederivat in seinem Molekül die durch die nachstehende Formel [I] dargestellte Struktur

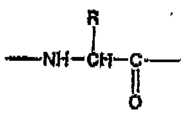

[I]

aufweist, wobei R eine mindestens eine basische funktionelle Gruppe enthaltende Gruppe bedeutet.

**4.** Retardierungspolymer für Aminosäurederivat nach einem der Ansprüche 1 bis 3, bei dem es sich bei dem Aminosäurederivat um eine basische Aminosäure handelt.

5. Retardierungspolymer für Aminosäurederivat nach einem der Ansprüche 1 bis 4, bei dem das Aminosäurederivat ein- oder mehrfach unter Arginin, Lysin und Histidin ausgewählt ist.

6. Retardierungspolymer für Aminosäurederivat nach einem der Ansprüche I bis 5, bei dem das saure Gruppen enthaltende Polymer bei 20 °C und 65 % Luftfeuchtigkeit eine hygroskopische Sättigung von 20 Gew.-% und mehr aufweist.

7. Retardierungspolymer für Aminosäurederivat nach einem der Ansprüche 1 bis 6, bei dem das saure Gruppen enthaltende Polymer eine Carboxylgruppe aufweist.

8. Retardierungspolymer für Aminosäurederivat nach einem der Ansprüche 1 bis 7, bei dem es sich bei dem saure Gruppen enthaltenden Polymer um ein Polymer eines Acrylsäuretyps handelt.

9. Retardierungspolymer für Aminosäurederivat nach einem der Ansprüche 1 bis 8, bei dem die vernetzte Struktur durch Reaktion der Nitrilgruppe mit einer Bindung des Typs Hydrazin entsteht.

10. Retardierungspolymer für Aminosäurederivat nach einem der Ansprüche 1 bis 8, bei der die vernetzte Struktur durch Copolymerisation eines vernetzenden Vinylmonomers entsteht.

11. Retardierungspolymer für Aminosäurederivat nach einem der Ansprüche 1 bis 10, bei dem das saure Gruppen enthaltende Polymer partikulär vorliegt.

12. Retardierungspolymer für Aminosäurederivat nach einem der Ansprüche 1 bis 10, bei dem das saure Gruppen enthaltende Polymer faserförmig vorliegt.

13. Verfahren zur Herstellung des Retardierungspolymers für Aminosäurederivat gemäß einem der Ansprüche 1 bis 12, bei dem man ein saure Gruppen enthaltendes Polymer mit einer Lösung von Aminosäurederivat versetzt und dann bei 40 bis 100 °C trocknet.

14. Kosmetikum, enthaltend das Retardierungspolymer für Aminosäurederivat gemäß Anspruch 11.

15. Fasergebilde, enthaltend das Retardierungspolymer für Aminosäurederivat gemäß Anspruch 11 oder 12.

16. Fasergebilde nach Anspruch 15, ausgewählt unter Unterwäsche, Bauchband, Korsett, Maske, Flandschuh, Socke, Strumpf, Schlafanzug, Bademantel, Handtuch, Matte und Bettwäsche.

17. Verfahren zur Herstellung des Fasergebildes gemäß Anspruch 15 oder 16, bei dem man ein ein saure Gruppen enthaltendes Polymer enthaltendes Fasergebilde mit einer Lösung von Aminosäurederivat versetzt und dann bei 40 bis 100 °C trocknet.

18. Verfahren zur Regenerierung eines Retardierungspolymers für Aminosäurederivat, bei dem man das Retardierungspolymer für Aminosäurederivat gemäß einem der Ansprüche 1 bis 12 oder das Fasergebilde gemäß Anspruch 15 oder 16, in welchem die damit verbundene Aminosäurederivatmenge gebrauchsbedingt reduziert ist, mit einer Lösung von Aminosäurederivat versetzt und dann trocknet.

## Revendications

1. Polymère à libération soutenue de dérivés d'acide aminé, où un polymère contenant un groupe acide est lié ioniquement à un dérivé d'acide aminé, où le polymère contenant un groupe acide présente une structure réticulée et où le taux d'élution ($\alpha$) du dérivé d'acide aminé lorsque le polymère est plongé dans un liquide sucré artificiel est de 10% ou plus et représente cinq fois ou plus de cinq fois le taux d'élution ($\beta$) du dérivé d'acide aminé lorsque le polymère est plongé dans de l'eau pure.

2. Polymère à libération soutenue de dérivés d'acide aminé selon la revendication 1, où le polymère peut être régénéré lorsqu'une solution du dérivé d'acide aminé est imprégnée dans celui-ci après la libération du dérivé d'acide aminé.

3. Polymère à libération soutenue d'un dérivé d'acide aminé selon les revendications 1 ou 2, où le dérivé d'acide aminé présente la structure telle que représentée par la formule [I] ci-dessous dans sa molécule.

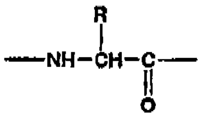

[ I ]

où R est un groupe présentant un ou plusieurs groupes fonctionnels basiques.

4. Polymère à libération soutenue d'un dérivé d'acide aminé selon l'une quelconque des revendications 1 à 3, où le dérivé d'acide aminé est un acide aminé basique.

5. Polymère à libération soutenue d'un dérivé d'acide aminé selon l'une quelconque des revendications 1 à 4, où le dérivé d'acide aminé est au moins un membre choisi dans le groupe constitué par l'arginine, la lysine et l'histidine.

6. Polymère à libération soutenue d'un dérivé d'acide aminé selon l'une quelconque des revendications 1 à 5, où le polymère contenant un groupe acide présente un degré hygroscopique saturé de 20% en poids ou plus dans la condition 20°C x 65% d'humidité relative.

7. Polymère à libération soutenue d'un dérivé d'acide aminé selon l'une quelconque des revendications 1 à 6, où le polymère contenant un groupe acide présente un groupe carboxyle.

8. Polymère à libération soutenue d'un dérivé d'acide aminé selon l'une quelconque des revendications 1 à 7, où le polymère contenant un groupe acide est un polymère du type acide acrylique.

9. Polymère à libération soutenue d'un dérivé d'acide aminé selon l'une quelconque des revendications 1 à 8, où la structure réticulée est formée par la réaction d'un groupe nitrile avec un composé de type hydrazine.

10. Polymère à libération soutenue d'un dérivé d'acide aminé selon l'une quelconque des revendications 1 à 8, où la structure réticulée est formée par copolymérisation d'un monomère de vinyle réticulant.

11. Polymère à libération soutenue d'un dérivé d'acide aminé selon l'une quelconque des revendications 1 à 10, où le polymère contenant un groupe acide est sous forme de particules.

12. Polymère à libération soutenue d'un dérivé d'acide aminé selon l'une quelconque des revendications 1 à 10, où le polymère contenant un groupe acide est fibreux.

13. Procédé pour la préparation du polymère à libération soutenue d'un dérivé d'acide aminé selon l'une quelconque des revendications 1 à 12, dans lequel une solution de dérivé d'acide aminé est ajoutée à un polymère contenant un groupe acide et le polymère est séché à 40 jusqu'à 100°C.

14. Cosmétique contenant le polymère à libération soutenue d'un dérivé d'acide aminé selon la revendication 11.

15. Structure fibreuse contenant le polymère à libération soutenue d'un dérivé d'acide aminé selon la revendication 11 ou 12.

16. Structure fibreuse selon la revendication 15, où la structure fibreuse est choisie parmi les sous-vêtements, une bande abdominale, un soutien-gorge, un masque, des gants, des bas, des chaussettes, un pyjama, un peignoir, une serviette, une carpette et un drap de lit.

17. Procédé pour la production de la structure fibreuse selon la revendication 15 ou 16, où une solution de dérivé d'acide aminé est ajoutée à un matériau à structure fibreuse qui contient un polymère contenant un groupe acide puis la structure fibreuse est séchée à 40 jusqu'à 100°C.

18. Procédé pour régénérer un polymère à libération soutenue d'un dérivé d'acide aminé, où une solution du dérivé d'acide aminé est ajoutée au polymère à libération soutenue d'un dérivé d'acide aminé selon l'une quelconque des

revendications 1 à 12 ou à la structure fibreuse selon la revendication 15 ou 16 dans lequel/laquelle la quantité de dérivé d'acide aminé qui y était liée a été diminuée suite à une utilisation, puis le polymère ou la structure fibreuse est séché(e).

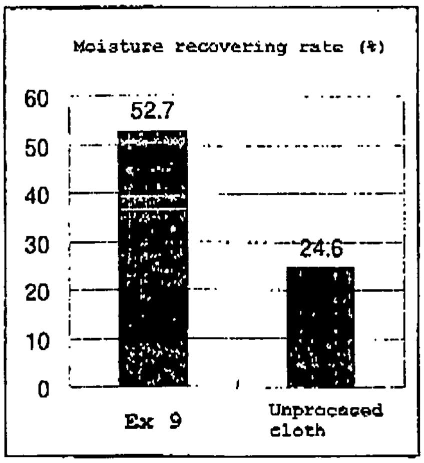
Moisture recovering rate (%)
60
50
40
30
20
10
0
52.7
24.6
Ex 9
Unprocessed cloth

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 8060547 A
- JP 2002013071 A
- JP 5295657 A
- JP 62161711 A
- JP 2002284619 A